# EUROPÄISCHE PATENTSCHRIFT

(11) **EP 0 672 403 B1**
(45) Veröffentlichungstag und Bekanntmachung des Hinweises auf die Patenterteilung: **19.04.2000**
(21) Anmeldenummer: 95100577.6
(22) Anmeldetag: 18.01.1995
(51) Int. Cl.: A61F 17/00

(54) **Nothilfepackung**
First-aid kit
Trousse de premier secours

(30) Priorität: 20.01.1994 DE 9400932 U
(43) Veröffentlichungstag der Anmeldung: 20.09.1995
(73) Patentinhaber: FRANZ KALFF GmbH, D-53881 Euskirchen (DE)
(72) Erfinder: Eichhorn, Wilfried, D-61273 Wehrheim (DE)
(74) Vertreter: Selting, Günther, Dipl.-Ing.

(56) Entgegenhaltungen:
- DE-C- 827 997
- DE-U- 7 002 608
- DE-U- 7 514 707
- DE-U- 8 002 838
- FR-A- 1 095 236
- US-A- 4 738 547

## Beschreibung

Die Erfindung bezieht sich auf eine Nothilfepackung mit Erste-Hilfe-Produkten, bestehend aus einer doppellagigen, flexiblen, länglichen Materialbahn, welche in durch kurze Zwischenabschnitte voneinander beabstandete, aufeinanderlegbare Querabschnitte unterteilt ist, die auf den beiden Materialbahn-Längshälften Taschen aufweisen, deren Öffnungen auf der Innenseite der Materialbahn liegen und parallel zu ihrer Längsmittellinie verlaufen.

Bei bekannten Nothilfepackungen dieser Art sind beispielsweise zwei Querabschnitte vorhanden, die aufeinanderklappbar sind und als unverschlossene Doppellagenpackung in den Hohlraum eines Kastens mit Klappdeckel einlegbar sind. Jeder Querabschnitt ist mit nur zwei Taschen versehen, von denen jede eine Materialbahn-Längshälfte besetzt. In jeder der verhältnismäßig großen Taschen sind die Erste-Hilfe-Produkte in beliebiger Sortierung lose übereinandergeschichtet enthalten, so daß Verbandsmaterial, Binden, Pflaster, Sicherheitsnadeln, Kreide, Scheren und dergleichen unübersichtlich untergebracht sind. Ein gewünschtes Produkt wird erst nach längerem Suchen in der Produktanhäufung einer Tasche gefunden. Die in einer Notsituation hinzukommende Aufregung erschwert die Auffindung bestimmter Produkte zusätzlich und es besteht auch die Gefahr, daß die Querabschnitte der unverschlossenen Nothilfepackung bei Herausnahme aus dem Kasten auseinanderklappen, so daß Produkte aus den Taschen heraus zu Boden fallen können. Die mangelnde Übersichtlichkeit behindert im übrigen die Überprüfung der Verfallsdaten der Produkte und gegebenenfalls ihren rechtzeitigen Austausch. Die Erste-Hilfe-Leistung kann sich dadurch verzögern, daß ein bestimmtes Produkt erst nach Durchsuchung sämtlicher Taschen gefunden wird und daß dann erst festgestellt wird, daß das Verfallsdatum überschritten und das Produkt nicht mehr brauchbar ist. Zusätzlich zu den Erste-Hilfe-Produkten stecken in den Taschen noch ein Merkblatt mit Instruktionen für So-fort-Maßnahmen und ein Inhaltsverzeichnis. Bei einer transparenten Materialbahn versperren diese Druckerzeugnisse die Sicht auf den Tascheninhalt.

Eine Nothilfepackung, von der der Oberbegriff des Patentanspruchs 1 ausgeht, ist bekannt aus DE-U-70 02 608. Diese bekannte Nothilfepackung besteht aus einer doppellagigen Folienbahn, deren Folienränder sich in der Längsmittelebene überlappen und unter Einschluß eines Aufreißstreifens miteinander verschweißt sind. Zu beiden Seiten des Aufreißstreifens sind Taschen gebildet. Die Taschen sind durch querlaufende Doppelschweißnähte, welche Zwischenabschnitte bilden, voneinander getrennt. Zur Aufnahme unterschiedlicher Gegenstände können die Taschen unterschiedliche Größe aufweisen. In einem solchen Fall haben die durch die Zwischenabschnitte voneinander getrennten Querabschnitte unterschiedliche Längen. Eine solche Nothilfepackung ermöglicht es, Taschen paarweise in gleicher Größe zu gestalten. Hierdurch kann zwar das Ordnungssystem verbessert werden, jedoch ist die Individualisierung der Taschengröße in bezug auf die Vielfalt der aufzunehmenden Gegenstände immer noch begrenzt.

Aus DE-U-80 02 838 ist eine Nothilfepackung bekannt, die auf einem Folienstreifen mehrere hintereinander angeordnete Taschen enthält. Die Taschenöffnungen verlaufen quer zur Längsrichtung des Folienstreifens und sie erstrecken sich jeweils über die gesamte Streifenbreite. Der Folienstreifen mit den gefüllten Taschen kann zu einer Packung gewickelt werden. Ein Endabschnitt des Folienstreifens ist als Überschlaglasche ausgebildet, die mit einem Befestigungsmittel an der gefalteten Pakung fixiert werden kann, um diese geschlossen zu halten. Auch hierbei sind in den relativ breiten längslaufenden Taschen Gegenstände oder Gruppen von Gegenständen unterzubringen, jedoch können diese Gegenstände beim Auseinanderfalten der Nothilfepackung verrutschen oder herausfallen.

Der Erfindung liegt die Aufgabe zugrunde, eine Nothilfepackung zu schaffen, die besser an die zu verpackenden Gegenstände angepaßt werden kann, um diese passend aufzunehmen, so daß eine einfache und sichere Handhabung gewährleistet ist.

Diese Aufgabe wird erfindungsgemäß mit den Merkmalen des Patentanspruchs 1 gelöst.

Die erfindungsgemäße Nothilfepackung weist unterschiedliche Querabschnitte auf. Einerseits sind Querabschnitte vorgesehen, bei denen die Länge der Taschen auf jeder der Materialbahn-Längshälften der Länge des zugehörigen Querabschnittes der Materialbahn gleich ist. Zusätzlich sind Querabschnitte vorgesehen, bei denen auf mindestens jeder einer der beiden Materialbahn-Längshälften mehrere Taschen nebeneinander angeordnet sind. Die Öffnungen der in einer der beiden Materialbahn-Längshälften angeordneten Taschen liegen den Öffnungen der in der anderen Materialbahn-Längshälfte angeordneten Taschen ohne gegenseitige Überlappung gegenüber.

Eine solche langgestreckte Aufnahmevorrichtung einer Nothilfepackung wird durch Übereinanderlegung der Querabschnitte zu einem Bündel geformt, das mit Hilfe eines Haftelementes an dem letzten Querabschnitt zusammengehalten wird. Die Anbringung unterschiedlich bemessener Taschen ermöglicht die Unterbringung der Erste-Hilfe-Produkte in passenden Taschen, so daß sie geordnet dargeboten werden und ohne Suchen entnommen werden können.

In den Taschen sind Gruppen von zusammengehörenden Produkten oder Einzelprodukte sortiert untergebracht, so daß selbst bei Übereinanderschichtung flacher Verpakkungen jeder Suchaufwand entfällt. Die Produkte sind in den Taschen festgelegt und ein Zusammenklappen bzw. Auseinanderklappen des Bündels der Nothilfepackung läßt sie nicht durcheinanderfallen. In den Taschen des letzten Querabschnittes, der eine Überschlaglasche bildet, können Druckerzeugnisse wie eine Anleitung zur Durchführung von Sofortmaßnahmen und ein Inhaltsverzeichnis untergebracht sein. Diese sind ggfs. nach Öffnung der Überschlaglasche zugänglich und können studiert werden, noch bevor das Bündel auseinandergeklappt worden ist. Die Übersichtlichkeit der Produkte in den Taschen erlaubt eine schnelle Überprüfung der Verfallsdaten und den rechtzeitigen Austausch von Produkten.

Die Nothilfepackung ist vorzugsweise so bemessen, daß sie in einen nach den geltenden Normvorschriften ausgebildeten, mit einem Deckel verschließbaren, steifen Kasten paßt, der als Schutzgehäuse dient, das nur einmal angeschafft werden muß und dessen z. B. überalterter Inhalt sich auf einfache Weise insgesamt preiswert erneuern läßt. Die gefüllten Querabschnitte sind infolge der Unverschiebbarkeit der Erste-Hilfe-Produkte in den Taschen recht flach und dieser Zustand bleibt auch nach mehrmaligem Auseinanderklappen der Nothilfepakkung erhalten, so daß diese ohne Zurechtrücken jeweils auf Anhieb in den Kasten paßt. Die Öffnungen der Taschen liegen vorzugsweise etwa auf der Längsmittellinie der Materialbahn, so daß die volle Breite der Querabschnitte für die Taschentiefe ausgenutzt wird und beim Aufschlagen des Bündels aus diesem nichts herausfallen kann.

In vorteilhafter Ausgestaltung der Erfindung kann mindestens ein Querabschnitt kürzer sein als andere Querabschnitte. Hierdurch läßt sich die Dicke des Bündels zur Berücksichtigung unterschiedlich dicker Erste-Hilfe-Produkte vergleichmäßigen und der Kastenhöhe angleichen.

In einer Lage der Materialbahn, vorzugsweise auf ihrer Innenseite, sind Löcher oder Schlitze ausgebildet, die paarweise eine Einsteckhalterung für einen länglichen Gegenstand bilden. Ein solcher länglicher Gegenstand ist beispielsweise eine Schere, die durch zwei Löcher oder Schlitze hindurchgesteckt in der Materialbahn gehalten ist. Nach Aufschlagen des Materialbahnbündels ist sie sofort griffbereit.

Vorzugsweise ist die doppellagige Materialbahn aus transparenter Kunststoffolie gebildet, die durch Nähte in Querabschnitte, Zwischenabschnitte und Taschengruppierungen unterteilt ist. Das Haftelement befindet sich bevorzugt am äußeren Querrand der Überschlaglasche und kann aus einem doppelseitig selbstklebenden Band, Bandabschnitten oder einem Klettband bzw. -fleck bestehen. Im letzteren Falle ist auf der Außenfläche der Materialbahn ein Gegenelement angebracht.

In der Zeichnung ist ein Ausführungsbeispiel der Erfindung schematisch dargestellt. Es zeigt:

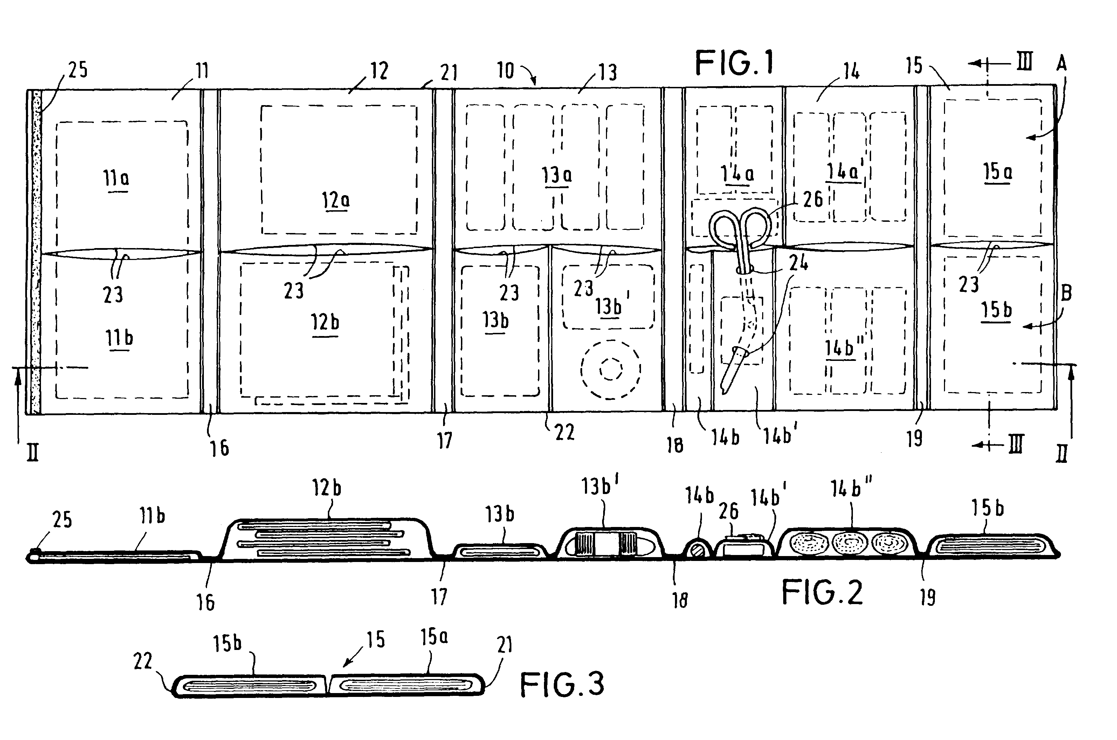

- Fig. 1: eine Draufsicht auf eine auseinandergeklappte Nothilfepackung mit Erste-Hilfe-Produkten;
- Fig. 2: einen Schnitt längs der Linie II - II in Fig. 1;
- Fig. 3: einen Schnitt längs der Linie III - III in Fig. 1; und
- Fig. 4: eine Ansicht der zusammengebündelten Nothilfepackung vor Einfügung in einen Kasten mit Klappdeckel.

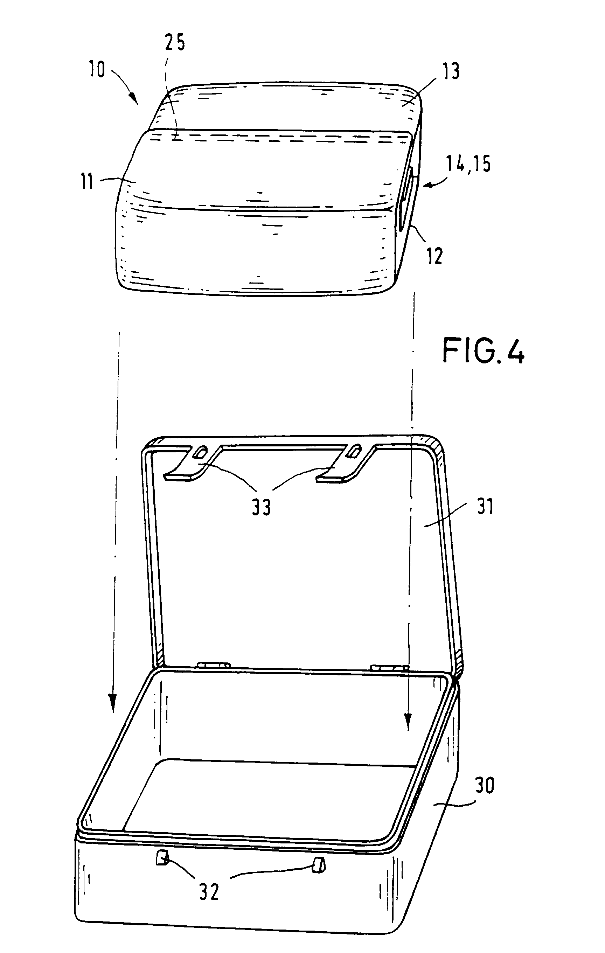

Eine Nothilfepackung 10 für gestrichelt angedeutete Erste-Hilfe-Produkte wie Mullbinden, Kompressen, Verbandtücher, Schnellverbände, Dreiecktücher, Heftpflaster sowie Sicherheitsnadeln und dergleichen, besteht im wesentlichen aus einer doppellagigen Materialbahn aus transparenter Kunststoffolie, die durch Schweißnähte in Querabschnitte 11, 12, 13, 14, 15 unterteilt ist. Die Schweißnähte sind so angesetzt, daß kurze Zwischenabschnitte 16, 17, 18, 19 zwischen den Querabschnitten 11 - 15 entstehen, die nach Art eines Buchrückens eine gewissen Parallelität der gefüllten Querabschnitte 11 - 15 in zusammengebündeltem Zustand der Materialbahn herbeiführen, so daß das in Fig. 4 zusammengeklappt gezeigte Bündel der Nothilfepackung 10 nicht rund, sondern etwa viereckig blockförmig ist.

Jeder Querabschnitt 11 - 15 ist auf den beiden Materialbahn-Längshälften A und B mit Taschen versehen, die in der Materialbahn-Längshälfte A mit dem Zusatz a und in der Materialbahn-Längshälfte B mit dem Zusatz b zu der Nummer des Querabschnittes bezeichnet sind. Der Deutlichkeit halber sind die Öffnungen aller Taschen mit 23 bezeichnet. Sie liegen auf der Längsmittellinie der Materialbahn. Die gezeichnete Wölbung der Öffnungsränder dient nur der Erkennbarkeit der Öffnungen 23 in der Darstellung. Die Taschen entstehen durch Umschlagen einer breiten Bahn entlang der Längsränder 21, 22 oder durch Aufschweißen von extra Bahnpartien auf eine Trägerbahn entlang der Längsränder 21, 22.

Die Querabschnitte 12 und 15 sind auf jeder Materialbahn-Längshälfte A und B mit je einer Tasche 12a, 12b bzw. 15a, 15b versehen, deren Länge der Länge des Querabschnittes 12 bzw. 15 entspricht. In den Taschen 12a, 12b stecken Verbandpäckchen und -tücher, die einlagig bzw. schuppenartig gestaffelt in den Taschen liegen. Die Taschen 15a, 15b nehmen passend bemessene Einzel-Tücherpackungen auf. In diesem Falle ergibt sich die gegenseitige Anpassung von Tasche und Inhalt dadurch, daß der Querabschnitt 15 so schmal ist, wie die Packungen.

Die Querabschnitte 13 und 14 haben gleiche Länge wie der Querabschnitt 12. In der Materialbahn-Längshälfte A des Querabschnittes 13 ist nur eine Tasche 13a entsprechender Länge vorgesehen, während der Querabschnitt 14 in der Materialbahn-Längshälfte A zwei Taschen 14a und 14a' aufweist. In der Materialbahn-Längshälfte B hat der Querabschnitt 13 zwei Taschen 13b und 13', während der Querabschnitt 14 in drei Taschen 14b, 14b' und 14b" unterteilt ist. Sämtliche Taschen a und b sind mit Erste-Hilfe-Produkten gefüllt, die entweder für sich alleine oder in Kombination mit anderen Produkten in die Taschen so passen, daß sie nicht verrutschen, wenn die Materialbahn zusammengeklappt oder auseinandergeklappt wird. Sie werden übersichtlich geordnet dargeboten. Der Querabschnitt 14 ist in der Materialbahn-Längshälfte B auf der Innenseite der Tasche 14b" mit zwei Löchern 24 versehen, die als Einsteckhalterung für eine Schere 26 dienen. Auch hierbei ist der erfindungswesentliche Gedanke verwirklicht, daß der Inhalt der Nothilfepakkung nicht verrutschen oder herausfallen kann und sofort griffbereit ist.

Der Querabschnitt 11 an dem einen Ende der Materialbahn dient als Überschlaglasche zum Verschluß der zusammengerollten oder zusammengefalteten Nothilfepackung 10. Als Verschlußorgan dient ein Haftelement 25, das bei dem gezeichneten Beispiel ein Selbstklebestreifen ist, der sich über die Breite der Materialbahn erstreckt und auf die Außenfläche des Bündels klebbar ist, so daß dieses sich nicht öffnet, bevor dies gewünscht wird. In den beiden Taschen 11a und 11b des Querabschnittes 11 stekken vorzugsweise eine Sofortmaßnahmen-Anleitung und ein Inhaltsverzeichnis, die einfach studierbar bzw. entnehmbar sind. Da sie in einem Endabschnitt der Materialbahn getrennt von den Erste-Hilfe-Produkten untergebracht sind, versperren sie nicht die Sicht auf die Produkte und sind beidseitig lesbar.

Das geschlossene Bündel der Nothilfepackung 10 paßt, wie Fig. 4 zeigt, in einen steifen Kasten 30, der durch einen Klappdeckel 31 verschließbar ist. Rastvorsprünge 32 an dem Unterteil des Kastens 30 wirken mit Schnapplaschen 33 zur Sicherung des Deckels 31 zusammen.

## Patentansprüche

1. Nothilfepackung mit Erste-Hilfe-Produkten, bestehend aus einer doppellagigen, flexiblen, länglichen Materialbahn, welche in mehr als zwei durch kurze Zwischenabschnitte (16,17,18,19) voneinander beabstandete aufeinanderlegbare Querabschnitte (11-15) unterteilt ist, die auf den beiden Materialbahn-Längshälften (A,B) Taschen (11a-15a,11b-15b) aufweisen, deren Öffnungen (23) auf der Innenseite der Materialbahn liegen und parallel zu ihrer Längsmittellinie verlaufen, wobei Querabschnitte (11,12,15) vorgesehen sind, bei denen die Länge der Taschen (a,b) auf jeder der beiden Materialbahn-Längshälften (A,B) der Länge des zugehörigen Querabschnittes gleich ist,
**dadurch gekennzeichnet,**
daß auch Querabschnitte (13,14) vorgesehen sind, bei denen auf mindestens einer der beiden Materialbahn-Längshälften (A,B) mehrere Taschen (a,b) nebeneinander angeordnet sind,
daß die Öffnungen (23) der in der einen Materialbahn-Längshälfte (A) angeordneten Taschen (11a-15a) den Öffnungen (23) der in der anderen Materialbahn-Längshälfte (B) angeordneten Taschen (11b-15b) ohne gegenseitige Überlappung gegenüberliegen, und
daß der letzte Querabschnitt (11) an einem Materialbahnende eine Überschlaglasche bildet.

2. Nothilfepackung nach Anspruch 1, dadurch gekennzeichnet, daß mindestens ein Querabschnitt (13) vorgesehen ist, bei dem auf einer Materialbahn-Längshälfte eine einzige Tasche (13a) und auf der anderen Materialbahn-Längshälfte mehrere Taschen (13b,13b') vorgesehen sind.

3. Nothilfepackung nach Anspruch 1 oder 2, dadurch gekennzeichnet, daß mindestens ein Querabschnitt (11,15) kürzer ist als andere Querabschnitte (11-14).

4. Nothilfepackung nach einem der Ansprüche 1 bis 3, dadurch gekennzeichnet, daß in einer Lage der Materialbahn Löcher (24) oder Schlitze ausgebildet sind, die paarweise eine Einsteckhalterung für einen länglichen Gegenstand (26) bilden.

5. Nothilfepackung nach einem der Ansprüche 1 bis 4, dadurch gekennzeichnet, daß die doppellagige Materialbahn aus transparenter Kunststoffolie gebildet ist, die durch Nähte in Querabschnitte (11-15), Zwischenabschnitte (16-19) und Taschengruppierungen (a,b) unterteilt ist und daß die Öffnungen (23) der Taschen (a,b) etwa auf der Längsmittellinie der Materialbahn liegen.

## Claims

1. First-aid package containing first-aid products, comprising a double-layer, flexible elongate sheet of material subdivided into more than two transverse sections (11 - 15) which are capable of being placed one upon the other and spaced apart from each other by short intermediate sections (16, 17, 18, 19), the transverse sections comprising on both longitudinal halves (A, B) pockets (11a - 15a, 11b - 15b) whose openings (23) are arranged on the inner side of the sheet of material and extend in parallel to its longitudinal centerline, wherein transverse sections (11, 12, 15) are provided where the length of the pockets (a, b) on each of the two longitudinal halves of the sheet of material (A, B) equals the length of the associated transverse section,
**characterized in that**
further transverse sections (13, 14) are provided where a plurality of pockets (a, b) are arranged side by side on at least one of the two longitudinal halves of the sheet of material (A, B),
the openings (23) of the pockets (11a - 15a) arranged on the one longitudinal half of the sheet of material (A) are located opposite the openings (23) of the pockets (11b - 15b) arranged on the other longitudinal half of the sheet of material (B) without overlapping each other, and
the last transverse section (11) forms a folding flap at one end of the sheet of material.

2. First-aid package according to claim 1, characterized in that at least one transverse section (13) is provided where on one longitudinal half of the sheet of material one single pocket (13a) is provided and on the other longitudinal half of the sheet of material a plurality of pockets (13b, 13b') are provided.

3. First-aid package according to claim 1 or 2, characterized in that at least one transverse section (11, 15) is shorter than the other transverse sections (11 - 14).

4. First-aid package according to one of claims 1 to 3, characterized in that in one layer of the sheet of material holes (24) or slots are provided which, in pairs, form a holder for insertion of an elongate object (26).

5. First-aid package according to one of claims 1 to 4, characterized in that the double-layer sheet of material is made of transparent plastic sheet which is subdivided by seams into transverse sections (11 - 15), intermediate sections (16 - 19) and groups of pockets (a, b), and that the openings (23) of the pockets (a, b) are arranged approximately on the longitudinal centerline of the sheet of material.

## Revendications

1. Trousse de premier secours comportant des produits pour premiers soins, constituée d'une bande de matière allongée et souple à deux couches, laquelle est subdivisée en plus de deux sections transversales (11 à 15), séparées les unes des autres par de courtes sections intermédiaires (16, 17, 18, 19) et susceptibles d'être superposées, ces sections présentant, sur les deux moitiés longitudinales de la bande de matière (A, B), des poches (11a à 15a, 11b à 15b) dont les ouvertures (23) se situent sur la face intérieure de la bande de matière et sont parallèles à leur axe de symétrie longitudinal, des sections transversales (11, 12, 15) étant prévues dans lesquelles, sur chacune des moitiés longitudinales de la bande de matière (A, B), la longueur des poches (a, b) est égale à la longueur de la section transversale correspondante,
caractérisée en ce qu'il est également prévu des sections transversales (13, 14) dans lesquelles, sur au moins l'une des deux moitiés longitudinales de bande de matière (A, B), plusieurs poches (a, b) sont disposées les unes à côté des autres,
en ce que les ouvertures (23) des poches (11a à 15a) disposées dans la première moitié longitudinale de bande de matière (A) se situent en face des ouvertures (23) des poches (11b à 15b) disposées dans l'autre moitié longitudinale de bande de matière (B) sans se chevaucher mutuellement, et
en ce que la dernière section transversale (11), située à une extrémité de la bande de matière, forme un rabat de fixation.

2. Trousse de premier secours selon la revendication 1, caractérisée en ce qu'il est prévu au moins une section transversale (13) dans laquelle, sur une moitié longitudinale de la bande de matière, une seule poche (13a) est prévue et, sur l'autre moitié longitudinale de la bande de matière, plusieurs poches (13b, 13b') sont prévues.

3. Trousse de premier secours selon l'une des revendications 1 ou 2, caractérisée en ce qu'au moins une section transversale (11, 15) est plus courte que d'autres sections transversales (11 à 14).

4. Trousse de premier secours selon l'une des revendications 1 à 3, caractérisée en ce que des trous (24) ou des fentes, qui forment deux à deux un support femelle destiné à un objet allongé (26), sont ménagés dans une couche de la bande de matière.

5. Trousse de premier secours selon l'une des revendications 1 à 4, caractérisée en ce que la bande de matière à deux couches est constituée de film de matière plastique transparente subdivisé, au moyen de soudures, en sections transversales (11 à 15), en sections intermédiaires (16 à 19) et en groupements de poches (a, b), et en ce que les ouvertures (23) des poches (a, b) se situent à peu près sur l'axe de symétrie longitudinal de la bande de matière.
